Europäisches Patentamt

**European Patent Office**

Office européen des brevets

(11) Publication number: **0 257 962**
**A2**

# EUROPEAN PATENT APPLICATION

(21) Application number: **87307286.2**

(22) Date of filing: **18.08.87**

(51) Int. Cl.⁴: **A 61 K 39/395**
**A 61 K 37/24, A 61 K 39/39**

(30) Priority: **29.08.86 US 902352**

(43) Date of publication of application:
**02.03.88 Bulletin 88/09**

(84) Designated Contracting States:
**AT BE CH DE ES FR GB GR IT LI NL SE**

(71) Applicant: **Becton, Dickinson and Company**
**One Becton Drive**
**Franklin Lakes New Jersey 07417-1880 (US)**

(72) Inventor: **Laniewr, Lewis L**
**1528 Fronzero Avenue, Santa Clara County**
**Los Altos California (US)**

**Phillips, Joseph H**
**305 Almaden Way San Mateo County**
**San Mateo California (US)**

(74) Representative: **Ruffles, Graham Keith et al**
**MARKS & CLERK 57-60 Lincoln's Inn Fields**
**London WC2A 3LS (GB)**

(54) **Method for treatment of peripheral blood to improve lymphokine activated killer immunotherapy.**

(57) A method for treatment of peripheral blood to improve the lymphokine activated killer cell (LAK) phenomenon. In the method, a sample of human peripheral blood is treated to recover a viable subpopulation of white blood cells consisting substantially of natural killer cells. The subpopulation of natural killer cells is incubated in the presence of a lymphokine to cause activation of the natural killer cells. The activated natural killer cells can then be used in adoptive immunotherapy.

## Description

METHOD FOR TREATMENT OF PERIPHERAL BLOOD TO IMPROVE LAK IMMUNOTHERAPY

Background of the Invention

Technical Field

The present invention is related to a method for treatment of peripheral blood to improve the lymphokine activated killer (LAK) cell phenomenon. More particularly, the present invention is related to improvements in adoptive immunotherapy as a treatment of certain disease types in humans and to a method of treating cancers and other immune diseases in humans using lymphokine activated natural killer cells.

Prior Art

Recently, attempts have been made to develop immunotherapies for the treatment of cancer based on stimulation of the host immune system to reject the tumor. The LAK cell phenomenon involves the incubation of normal murine splenocytes or human peripheral blood lymphocytes in a lymphokine, which results in the generation of lymphoid cells with the ability to lyse fresh, non-cultured, natural killer cell (NK)-resistant cancer cells in short term chromium release assays. LAK cells appear to be relatively specific for neoplastic cells and are not efficiently lytic to fresh normal lymphocytes or liver or lung cells.

More recently, adoptive immunotherapy of cancer using lymphoid cells activated with interleukin-2 lymphokine (IL-2) has been disclosed; Rosenberg S.A. The Adoptive Immunotherapy Of Cancer Using The Transfer Of Activated Lymphoid Cells And Interluekin-2. Seminars In Oncology, Vol. 13, No. 2 (June), 1986: PP200-206; Resenberg S.A., Immunotherapy Of Cancer By Systemic Administration of Lymphoid Cells Plus Interleukin-2, Journal Of Biological Response Modifiers 3:501-511, 1984; Rosenberg S.A. et al, Observations On The Systemic Administration Of Autologous Lymphokine - Activated Killer Cells And Recombiant Interleukin-2 To Patients With Metastatic Cancer, The New England Journal Of Medicine, Vo. 313, No. 23, pp 1485-1492, 1985.

Adoptive immunotherapy is defined as the transfer to the tumor-bearing host of active immunologic reagents, such as cells with anti-tumor reactivity that can mediate directly or indirectly, anti-tumor effects. The adoptive immunotherapy described in the Rosenberg references involves the use of LAK cells in combination with the administration of IL-2. The LAK cells are prepared by a process involving the leukapheresis of peripheral blood obtained from a patient followed by separation of the mononuclear cells from the granulcytes by Ficoll-Hypaque density gradient separation. The mononuclear cells, including B-cells, T-cells, null-cells, monocytes and natural killer (NK) cells are then incubated with IL-2 for a period of three-four days. The resulting LAK cells are centrifuged, resuspended in infusion medium and filtered through sterile Nytex (110 mesh) and transferred to a transfer pack. The LAK cells are administered to a patient on a timed interval basis with intervening administration of IL-2 to aid in the activation of cells within the patient. The adoptive immunotherapy treatment described in the Rosenberg references resulted in the prevalence of numerous toxic effects. The most severe toxicity resulted from the administration of IL-2.

The adoptive immunotherapy described in the Rosenberg references utilized the entire range of lymphocyte cells for the incubation treatment with IL-2. There is no recognition in the Rosenberg references that a particular sub-population of lymphocyte cells is responsible for the LAK phenomenon. It has been thought, however, that actual LAK cells are a subpopulation of T-cells and/or null cells. It is recognized in the Rosenberg references that improvements are required in the performance of the adoptive immunotherapy described in the references before it can be more widely applied.

The discovery of the present invention is that the subclass of lymphocyte cells in peripheral blood identified as natural killer cells are the progenitors of the LAK cells and, in fact, are much more potent when treated with IL-2 when separated from the other mononuclear cells than when the natural killer cells are subjected to IL-2 incubation in the presence of other mononuclear cells, such as T-cells and monocytes.

Summary of the Invention.

It is an object of the present invention to provide a method for the treatment of peripheral blood to provide more potent LAK cells.

It is another object of the present invention to provide human peripheral blood natural killer cells activated with a lymphokine, said activated natural killer cells, when administered to humans, being selectively capable of destroying tumor cells.

Other objects and advantages of the present invention will become more apparent from the following detailed description of the invention.

Description of the Drawings.

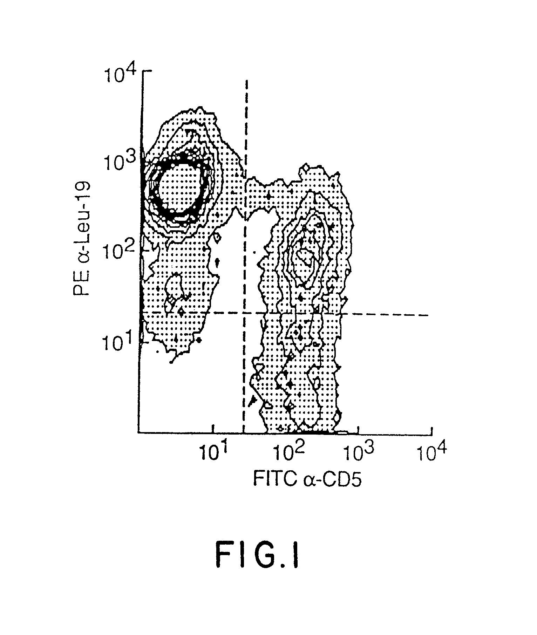

FIGURE 1. Coexpression of CD5 and Leu 19 on low bouyant density lymphocytes. Low bouyant density lymphocytes were stained with FITC-conjugated anti-Leu-1 (CD5) and biotinconjugated anti-Leu-19, followed by PE-conjugated streptavidin. Cells were analyzed by flow cytometry, and data were displayed as contour plots of FITC and PE fluorescence. The contour map was divided into quadrants to represent: cells stained only with PE (upper left) cells stained with FITC and PE (upper right); cells stained only with FITC (lower right) and unstained cells (lower left). In the control sample (cells stained with FITC and PE-conjugated IgG control antibodies), 98% of lymphocytes were in the lower right quadrant. In subsequent figures, CD5–, Leu-19+ lymphocytes are referred to as NK cells, CD5+, Leu-19+ lymphocytes are referred to as Leu-19+ T cells, and CD5+, Leu-19– lymphocytes are referred to as Leu-19– T cells.

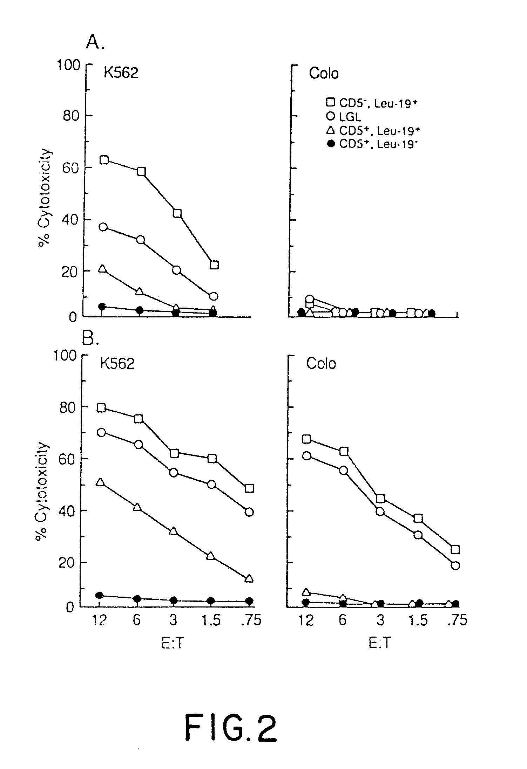

Figure 2. Cytotoxicity mediated by rIL-2-cultured low bouyant density lymphocyte subsets. A: Freshly isolated low bouyant density lymphocytes (LGL:O), Leu-19+ T cells ( Δ ), Leu-19– T cells ( ● ), and NK cells ( □ ) were assayed for cytotoxic activity against K562 (left) and COLO (right) before culture. B: Low bouyant density lymphocytes were cultured in medium containing rIL-2 for 7 days. Lymphocytes were harvested and stained with mAbs, as described in Fig. 1. rIL-2-activated Leu-19+ t cells, Leu 19– T cells, and NK cells were isolated using the FACS and tested for cytotoxicity against K562 (left) and COLO (right).

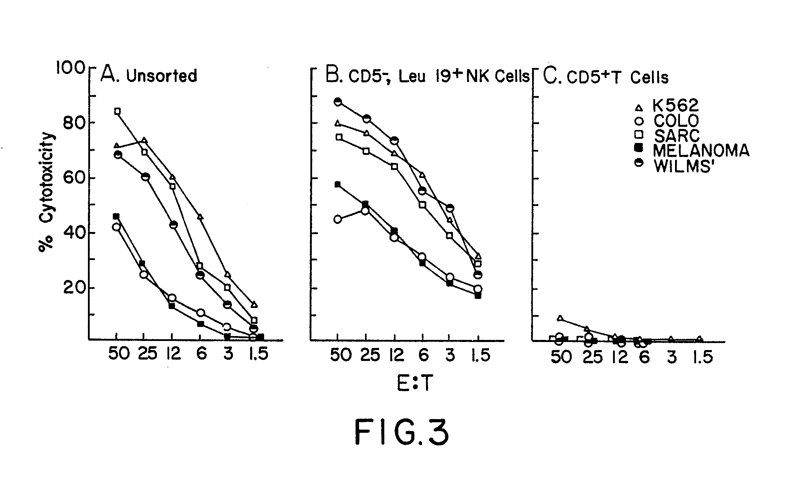

Figure 3. Cytotoxicity against freshly isolated tumor cells mediated by low bouyant density lymphocyte subsets. Low bouyant density lymphocytes were cultured in medium containing rIL-2 for 7 d. Lymphocytes were harvested and stained with mAbs, as described in Fig. 1. rIL-2 activated unsorted cells (A), CD5–, Leu-19+ NK cells (B), and T cells (pool of CD5+, Leu-19– and CD5+, Leu-19+) (C) were tested for cytotoxicity against K562 ( Δ ), COLO (O), a fresh melanoma tumor ( ■ ), fresh sarcoma tumor ( □ ) and fresh Wilms' tumor ( ◐ ).

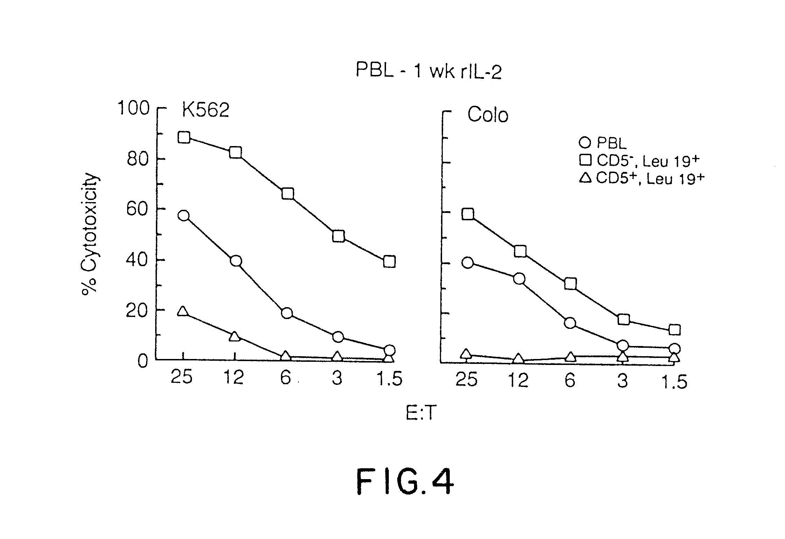

Figure 4. Cytotoxicity mediated by rIL-2 cultured peripheral blood mononuclear cell subsets. Peripheral blood mononuclear cells (O) isolated from Ficoll/Hypaque gradients were cultured in medium containing rIL-2 for 7 d. Lymphocytes were harvested and stained with mAbs, as described in Fig. 1. Leu-19+ cells ( Δ ) and NK cells ( □ ) were isolated using the FACS and tested for cytotoxicity against K562 (left) and COLO (right).

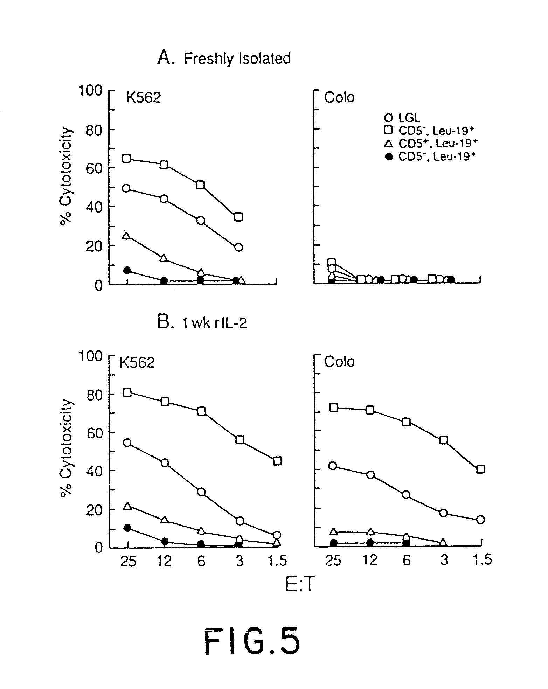

Figure 5. Precursors of rIL-2-activated cytotoxic cells. A: Freshly isolated low bouyant density lymphocytes (O) were stained with mAbs, as described in Fig. 1. Leu-19+ T cells ( Δ ), Leu-19– T cells ( ● ) and NK cells ( □ ) were isolated using the FACS and assayed immediately for cytotoxic activity against K562 (left) and COLO (right). B: Leu-19+ T cells, Leu-19– T cells and NK cells were isolated using the FACS and placed in culture with rIL-2. After 7 d, cells were harvested and assayed for cytotoxicity against K562 (left) and COLO (right).

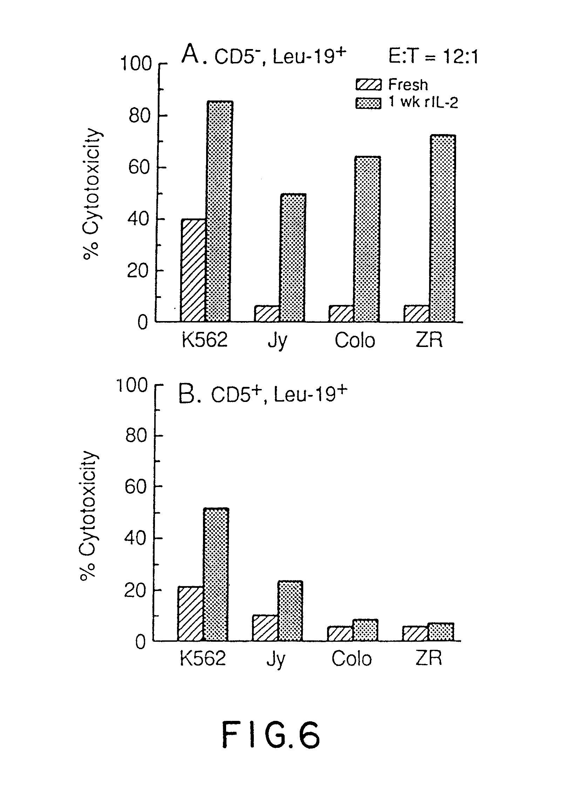

Figure 6. Cytotoxicity mediated by rIL-2 activated NK cells and Leu-19+ T cells. NK cells (A) and Leu-19+ T cells (B) were isolated using the FACS and placed in culture with rIL-2 for 7 d. Freshly isolated cells and rIL-2 activated cells were assayed for cytotoxicity against K562, JY, COLO, and ZR. E/T ratio is 12:1.

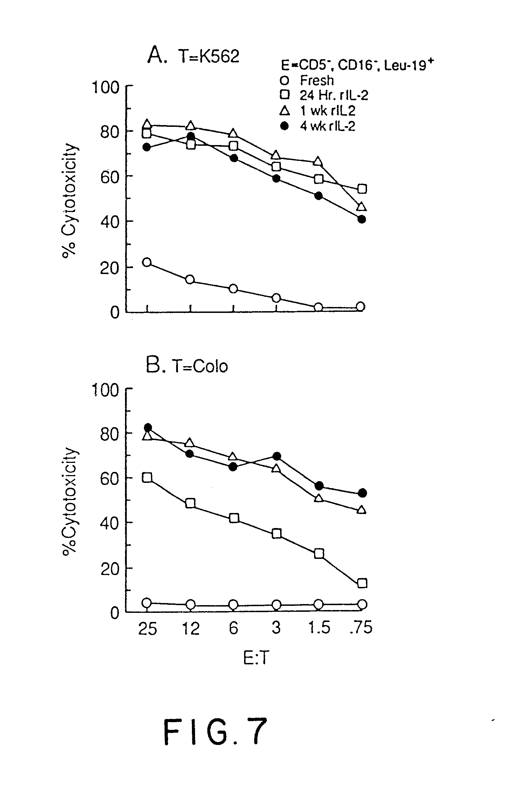

Figure 7. Cytotoxicity mediated by rIL-2-activated CD3–, CD 16–, Leu-19+ cells. Freshly isolated low bouyant density lymphocytes were stained with mAbs, as described in the text. CD3–, CD-16–, Leu-19+ lymphocytes were isolated using the FACS and placed in culture with rIL-2 for 24 h ( □ ) ), 1 wk ( Δ ) and 4 wk ( ● ). Freshly isolated (O) and rIL-2-activated cells were assayed for cytotoxicity against K562 (A) and COLO (B).

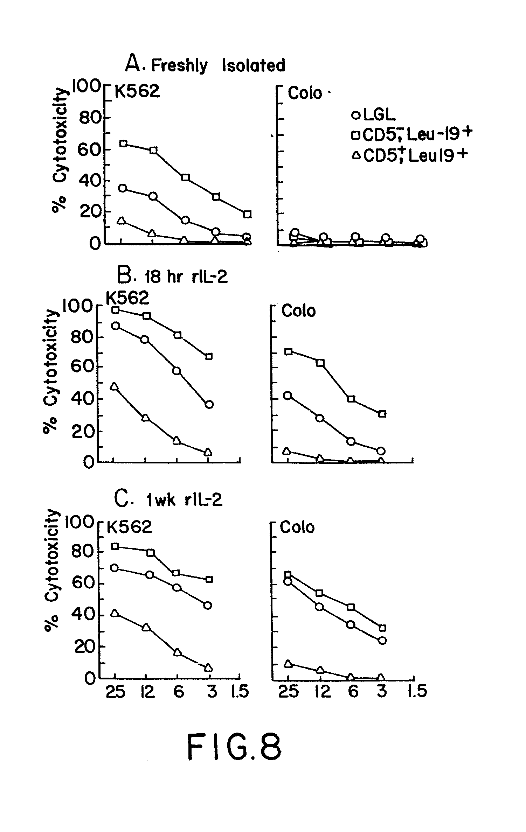

Figure 8. Kinetics of rIL-2-activated cytotoxicity. Freshly isolated low bouyant density lymphocytes (O) were stained with mAbs, as described in Fig. 1. Leu-19+ T cells ( Δ ) and NK cells ( □ ) were isolated using the FACS and placed in culture with rIL-2. At the indicated time points (A, freshly isolated; B, 18 h; C 1 wk), cells were harvested and assayed for cytotoxicity against K562 (left) and COLO (right).

Glossary

As used herein the following terms have the indicated meanings:
Percoll - a density gradient media commercially available through Pharmacia Five Chemicals AB.
Pan-T monoclonal antibody - A monoclonal antibody that reacts with substantially all T lymphocytes.
HBSS - Hank' balanced salt solution, an isotonic salt solution.
PBS - phosphat buffered saline, an isotonic salt solution.
FCS - fetal calf serum.
mAB - monoclonal antibody
RPM1-1640 - a commercially available culture medium for lymphocytes.
CD designation for mABs - World Health Organization recognized nomenclature for human leukocyte differentiation antigens. Anti-leu 1 aand anti-leu 11 react with the CD5 and CD16 antigens, respectively.
rIL-2 - recombinant interleukin-2.
MHC - major histocompatibility complex, class I antigens, such as HLA in humans.

Detailed Description Of The Invention

The objects and advantages of the present invention are achieved by utilizing a method for treatment of peripheral blood wherein natural killer cells are separated from the other components of the blood and are activated with a lymphokine prior to administration of the activated natural killer cells in a suitable pharmaceutically acceptable carrier to humans suffering from an immune-disfunction condition or cancer. A preferred lymphokine suitable for activation of the separated natural killer cells is Interleukin-2 (IL-2). IL-2 can be obtained from any suitable source including the use of recombinant DNA techniques for producing IL-2. The lymphokine activated natural killer cells possess anti-tumor activity and may be administered in doses smaller than heretofore associated with adoptive immuno-therapy techniques. The lymphokine activated natural killer cells are referred to herein as LANK cells.

The natural killer cell sub-population of white blood cells can be harvested by any suitable method. For example, monoclonal antibodies, identified as anti-leu 11 or anti-leu 19, manufactured by Becton, Dickinson

3

Monoclonal Center, Mountain View, CA) can be used to extract natural killer cells from the other white blood cell components of peripheral blood by direct or indirect methods. In a direct method the monoclonal antibodies selective for natural killer cells can be adhered to a column and the peripheral blood can then be passed through the column. The natural killer cells react with the monoclonal antibody and are removed from the peripheral blood. The natural killer cells can then be recovered from the column by elution.

It is preferred, however, to harvest the natural killer cell subpopulation by a method that does not involve direct attachment of the natural killer cells to a monoclonal antibody. In particular, since the serum and erythrocytes of the peripheral blood usually needs to be returned to a patient while the white blood cells are being treated with a lymphokine, it is preferred to use leukophoresis to separate the white blood cell population from the blood serum and erythrocytes so that the blood serum and erythrocytes can be immediately returned to a patient. After removal of the white blood cells from the blood serum, the granulocytes are separated by a treatment. The Immunological analysis of blood generally requires the isolation and separation of the lymphocytes for detailed analysis. In general, two distinct methods are known for the separation of lymphocytes from other blood cells. The first of these methods involves bouyant density centrifugation of cells through a particular newtonian fluid. The most commonly used fluid is known as Ficoll-Pague, a water soluble liquid having a specific gravity of 1.077 g/cc, which is marketed by Pharmacia Fine Chemicals AB, Upsala, Sweden. The second of these methods utilizes a non-newtonian, water-insoluble, thiotropic, gel-like substance which establishes a continuous semi-rigid gel-like seal across the interior of a container between the lighter phase containing the lymphocytes and a heavier phase containing the erythrocytes and granulocytes.

The Ficoll-Paque method contemplates the following general steps:

(1) A predetermined amount of Ficoll-Paque is dispensed into the bottom of a container suitable for centrifugation, such as a test tube;

(2) A sample of whole or diluted blood is carefully pipetted onto the surface of the Ficoll-Paque

(3) The Ficoll-Paque/blood preparation is then centrifuged at about 400g for about 30 minutes to provide a layered separation of blood constituents into a top lighter phase containing a mononuclear cell layer including the lymphocytes and a bottom heavier phase containing the erythrocytes and granulocytes which pass into and/or through the Ficoll-Paque;

(4) An upper plasma layer is removed by pipetting, leaving behind a lymphocyte layer on the surface of the Ficoll-Paque.

After Ficoll-Paque® treatment or other suitable treatment to remove granulocytes, the majority of the monocyte cells are removed from the balance of the white blood cell population by sequential adherence to plastic tissue culture flasks. The residual monocyte cells and B cells are then removed by adherence to the fibrous nylon wool. The portion of cells which pass through the fibrous nylon wool column comprises substantially all T-cells and all natural killer cells and may include some null-cells. This portion of white blood cells is then subjected to density gradient centrigation treatment, such as described in Timonen, T. et al, Isolation of Human Natural Killer Cells by Density Gradient Centriguation, J. Immunol. Methods, 36: pp 185-=291 (1980). The cells are resuspended in 30% in volume of percoll in HBSS and layered on top of a cushion of 40% in volumn of Percoll in a conical centrifuge tube. The tube is centrifuged at about 2000 RPM for about 40 minutes. Large granular lymphocytes (LGL) which comprises a low buoyant density fraction remain at the interface of the Percoll gradient. The large granular lymphocytes harvested from this fraction contains substantially about 60% natural killer cells and about 40% of T-cells, depending upon the blood donor.

The LGL fraction recovered from the Percoll gradient centrifugation treatment is then subjected to treatment with a Pan-T monoclonal antibody to remove the T-cells from the fraction. The Pan-T antibody, such as anti-Leu 1, manufactured by Becton, Dickinson Monoclonal Center, is mixed with the cells to cause reaction of the monoclonal antibody with the T-cells. The cells can then be passed through a column having an anti-immunoglobulin antibody to cause adherence of the reacted T-cells with the anti-Leu 1. In another method, the Pan-T monoclonal antibody can be coated onto a column and the fraction flowed through the column to cause reaction of the T-cells with the Pan-T monoclonal antibody coated onto the column. The fraction that flows thru the column contains the natural killer cells.

After recovery of the natural killer cells, the natural killer cells are incubated with a lymphokine, such as IL-2 for a period of 12 to 48 hours to provide lymphokine activated natural killer cells (LANK). The LANK cells can then be administered to a patient at a level much lower than the LAK cells heretofore provided by treatment of the whole population of white blood cells.

EXAMPLE

Human Peripheral Blood Leukocytes.

Human peripheral blood from normal, random donors was obtained from the Stanford Blood center, Palo Alto, CA. The mononuclear cells were isolated using Ficoll/Hypaque. After passage through nylon wool, nonadherent lymphocytes were fractionated by centrifugation on discontinuous gradients consisting of 30% and 40% Percoll (Pharmacia Fine Chemicals, Piscataway, NJ) in PBS containing 10% FCS. Low bouyant density and high bouyant density lymphocytes were isolated from the interface and bottom of the Percoll gradients, respectively. Total cell recovery was 85%. 25% of lymphocytes were in the low bouyant density fraction, depending on the individual donor. The population of lymphocytes in the interface fraction were highly enriched for cells with large granular lymphocyte (LGL) morphology.

Monoclonal Antibodies.
Leu series mAbs were prepared at the Becton Dickinson Monoclonal Center; Mountain view, California. The CD Nomenclature of the differentiation antigens identified by these mAbs is summarized in Table 1.

## TABLE 1

## CD Nomenclosure for Differentiation Antigens

| CD cluster | mAb | Major cell reactivity |
|---|---|---|
| CD3 | Leu-4 | T cells (antigen receptor complex) |
| CD4 | Leu-3 | T subset and monocytes |
| ·CD5 | Leu-1 | T cells and B cell subset |
| CD8 | Leu-2 | T subset and NK subset (Fc receptor) |
| CD16 | Leu-11 | NK cells and neutrophils (Fc receptor) |
| ND | Leu-19(NKH-I) | NK cells and T subset |

Anti-Leu 11 reacts with CD16, the Fc receptor for IgG expressed on NK cells and neutrophils. Anti-Leu-19 reacts with the Leu-19 (NKH-I) antigen, protein of approximately 220 KD of unknown function expressed on NK cells and a subset of T lymphocytes.

Immunofluorescence and Flow Cytometry.
Methods of immunofluorescence, flow cytometry, and data analysis have been described, Lanier, L. L., A. M. Le, J. H. Phillips, N. L. Warner, and G. F. Babcock. 1983. Subpopulations of human natural killer cells defined by expression of the Leu-7 (HNK-1) and Leu-11 (NK-15) antigens. J. Immunol. 131:1789, Parks, D. R., L. L. Lanier, and L. A. Herzenberg. 1986. Flow cytometry and fluorescence activated cell sorting (FACS). In Handbook of Experimental Immunology, 4th edition, D. M. Weir, L. A. Herzenberg, C. C. Blackwell, and L. A. Herzenberg, editors. Blackwell Publications, Ltd., Edinburgh, United Kingdom. In press, Phillips, J. H., and L. L. Lanier. 1985. A model for the differentiation of human natural killer cells. Studies on the in vitro activation of Leu-11+ granular lymphocytes with a natural killer-sensitive tumor cell, K562. J. Exp. Med. 161:1464. A FACS 440 flow cytometer and Consort 40 data analysis system (Becton Dickinson Immunocytometry Systems, Mountain View, CA) were used. Fluorochrome-conjugated, isotype-matched mAbs that do not react with human leukocytes were used to control for nonspecific binding in the immunofluorescence assays.

Cell Lines.
All tumor cell lines were obtained from the American Type Culture Collection, Rockville, MD and were tested monthly to ensure against mycoplasma infection. Freshly isolated human tumors were provided by Drs. Anthony Rayner and Brett Gemlo (Department of Surgery, University of California Medical School, San Francisco, CA).

Cytotoxicity Assays.
Cr-labeled tumor cells were used as targets in a 4-h radioisotope release assay.

Lymphocyte Activation.
Lymphocytes ($10^6$ cells/ml) were cultured in RPMI-1640 (M.A. Bioproducts, Walkersville, MD) containing 5% horse serum (JR Scientific, Woodland, CA), 1 mM glutamine (Gibco Laboratories, Grand Island, NY), and 100 ug/ml gentamicin (Gibco). rIL-2 was added to the cultures at 1,000 U/ml. Preliminary studies established that this concentration of rIL-2 induced optimal cytotoxicity, although lower concentrations were sufficient to initiate the response.

Characterization of Effector Cells.

It has been shown that two distinct subsets of peripheral blood lymphocytes can lyse NK-sensitive tumor targets without deliberate immunization or MHC-restriction. These cytotoxic subsets were identified by two-color immunofluorescence using antibodies against CD3, a component of the T cell antigen receptor and the Leu-19 (NKH-1) antigen. Both CD3−, Leu-19+ NK cells and the CD3+, Leu-19+ NK cells and the CD3+, Leu-19+ T cells isolated directly from peripheral blood mediated non-MHC restricted cytotoxicity). Within the CD3+, Leu-19+ cell subset, most cells expressed the CD2, CD5, and CD8 differentiation regions, but did not express CD 16 (10). In contrast, 90% of the CD3−,Leu-19+ NK cell subset coexpressed CD16, and none expressed the pan T cell antigen CD5. The minor population of CD3−, CD16−, Leu-19+ lymphocytes also mediated non-MHC restricted cytotoxicity, and were phenotypically identical to CD3−, CD16+, Leu-19+ NK cells, with the exception of lacking CD 16 expression.

In the present studies, the relative contribution of NK cells (CD−, Leu-19+), and T cells (CD3+, Leu-19−) to the LAK phenomenon were examined. Prior studies indicated that precursors of the LAK phenomenon were low bouyant density lymphocytes that comigrated, on Percoll gradients, with NK cells. Therefore, low bouyant density lymphocytes (designated LGL) from normal peripheral blood were cultured in medium containing rIL-2. After 7 days, cells were harvested and stained with FITC-conjugated anti-Leu-1 (CD5) and biotin-conjugated anti-Leu-19, followed by PE (phycoerythrin)-conjugated streptavidin (Fig. 1). anti-CD5 was used to identify and isolate T cells, since antibodies against CD3 have been shown to either inhibit or induce T cell-mediated cytotoxicity. NK cells (CD5−, Leu-19+, comprising 55% of total low bouyant density lymphocytes), Leu-19+ T cells (CD5+, Leu-19+, 24%), and Leu-19− T cells (CD5+, Leu-19−, 14%) were isolated by cell sorting using a FACS. Reanalysis indicated 95% purity in all populations. These rIL-2 activated cells were then tested for cytotoxicity against the NK-sensitive tumor cell line, K562, as well as an NK-resistant colon carcinoma cell line (COLO) (Fig. 2B). Both the rIL-2 activated NK cells and Leu-19+ T cells lysed K562. However, significant lysis of the solid tumor cell line was predominately mediated by rIL-2-activated NK cells. rIL-2-activated Leu-19+ T cells showed only minimal cytotoxic activity against COLO, while Leu-19− T cells totally lacked cytotoxic function against either K562 or COLO (Fig. 2B). For comparison, we also isolated NK cells, Leu-19+ T cells, and Leu-19− T cells from the low bouyant density lymphocytes population, and tested these freshly isolated cells for cytotoxicity before culture in rIL-2. As shown in Fig. 2A, freshly isolated NK cells efficiently lysed K562, but did not lyse COLO. Freshly isolated Leu-19+ T cells were not cytotoxic. Essentially identical results were obtained with lymphocytes isolated from three different blood donors.

Cytotoxicity against freshly isolated human tumors was also mediated exclusively by IL-2-activated NK cells, as shown in Fig. 3. In these experiments, low bouyant density lymphocytes were cultured in rIL-2, and the NK cell (CD5−, Leu-19+) and T cell (CD5+, Leu-19− and CD5+, Leu-19−) subsets were isolated using the FACS. rIL-2-activated NK cells and T cells were assayed for cytotoxicity against K562, the colon carcinoma cell line (COLO), freshly isolated melanoma tumor cells, freshly isolated Wilms' tumor cells, and freshly isolated sarcoma tumor cells. Freshly isolated tumors, as well as the COLO cell line, were efficiently killed by rIL-2-activated NK cells, but not rIL-2-cultured T cells (fig. 3). As seen previously, rIL-2-cultured T cells mediated low levels of cytotoxicity against K562. It should be noted that the magnitude of cytotoxicity against the three freshly isolated tumors was equivalent to the activity observed against COLO. COLO and the freshly isolated tumor cells were completely resistant to lysis mediated by unactivated peripheral blood NK cells (not shown). Since cytotoxicity against COLO and the fresh tumor cells was equivalent, COLO was used for most subsequent experiments.

To exclude the possibility that Percoll fractionation biased these results, unseparated peripheral blood mononuclear cells were also cultured in rIL-2. After 7 days of culture, cells were stained with FITC-conjugated streptavidin. Leu-19+ T cells (comprising 21% of total lymphocytes), Leu-19− T cells (66%), and NK cells (8%) were isolated to 95% purity using the FACS. Again, lysis of the NK-insensitive colon cell line, COLO, was predominately mediated by rIL-2-activated NK cells (Fig. 4). Although peripheral blood rIL-2-activated Leu-19+ T cells showed low levels of killing against K562, these T cells killed neither K562 nor COLO.

Characterization of Precursor Cells.

Low bouyant density peripheral blood lymphocytes, enriched on Percoll gradients, were stained with FITC-conjugated anti-Leu 1 (CD5) and biotin-conjugated anti-Leu-19, followed by PE-streptavidin. Leu-19+ T cells, Leu-19+ T cells, Leu-19− T cells, and NK cells were isolated to 95% purity using the FACS, and were placed in culture in medium containing rIL-2. After 5-7 days, lymphocytes were harvested and examined for antigenic phenotype and cytotoxic function. After culture in rIL-2, 95% of the Leu-19+ T lymphocytes retained the phenotype CD3+, CD5+, Leu-19+. Likewise, Leu-19− T lymphocytes remained CD3+, CD5+, Leu-19−. 95% of rIL-2-cultured NK cells maintained the phenotype CD3−, CD5−, Leu-19+, and as observed before culture, 90% expressed the CD16 antigen. There was no evidence that NK cells ever acquired pan T cell antigens as a consequence of culture in rIL-2 or that T cells lost CD3 expression after culture.

The cytotoxic activity of these subsets was tested before culture (Fig. 5A) or after culture in rIL-2 for 7 days (Fig. 5B). Again, the rIL-2 cultured NK cells were potent mediators of lysis against both K562 and COLO; whereas the freshly isolated NK cells lysed K562, but not COLO (Fig. 5). In contrast, freshly isolated and rIL-2-activated Leu-19+ T cells lysed K562, but showed only minimal cytotoxicity against COLO. In this individual, culture in rIL-2 did not significantly augment the cytotoxicity of Leu-19+ T cells against K562; however, in several other individuals (e.g., Figs. 6 and 8), rIL-2-enhanced cytotoxicity against K562 was

6

observed, but not COLO. Non-MHC restricted cytotoxic lymphocytes were not generated by culture of Leu-19– T cells in rIL-2.

To further examine the cytotoxic potential of these cells, NK cells and Leu-19+ T cells were isolated from the low bouyant density lymphocyte population, were cultured for 1 wk. in rIL-2, and were then assayed for cytolytic activity against K562, COLO, ZR (an NK-resistant breast carcinoma cell line), JY (an NK-resistant B lymphoblastoid cell line), and normal allogeneic lymphocytes (Fig. 6). Freshly isolated NK cells and Leu-19+ T cells lysed K562, although NK cells were more effective effectors. Against the hematopoietic cell targets (K562 and JY), rIL-2 enhanced the cytotoxicity of both the NK cells and the Leu-19+ T cells. In contrast, only the NK cells showed substantial rIL-2-induced cytotoxicity against the solid tumor cell lines.

These results unequivocally demonstrate that rIL-2 provides the only signal necessary for NK cells to lyse solid tumor cell targets. T cells, accessory cells, or additional factors are apparently not required. Furthermore, these studies show that NK cells with the ability to efficiently lyse NK-insensitive solid tumor cell targets arise from a precursor population with the phenotype CD5–, Leu-19+. More extensive immunophenotyping has shown that 90% of these precursor cells coexpress CD16, and all lack CD3. It has been recently reported that this CD3–, CD16+ NK cell population does not rearrange T cell antigen receptor B genes, Lanier, L. L., S. Cwirla, N. Federspiel, and J. H. Phillips. 1986. Human peripheral blood natural killer cells isolated from peripheral blood do not rearrange the T cell antigen receptor B-chain genes. J. Exp. Med. 163:209, suggesting that NK cells are distinct in origin from the T cell lineage and do not use the T cell antigen receptor for target recognition.

Within the CD3–, Leu-19+ NK cell subset, 10% of these cells lack expression of CD16. Freshly isolated CD3–, CD16–, Leu-19+ lymphocytes killed NK-sensitive tumor cell targets, but not NK-insensitive targets (10). To determine whether this population responds to rIL-2, low bouyant density lymphocytes were stained with a combination of FITC-conjugated anti-Leu-4(CD3), FITC-conjugated anti-Leu-11 (CD16), and biotin-conjugated anti-Leu-19, followed by PE-conjugated streptavidin. Lymphocytes displaying only red (PE) immunofluorescence would therefore possess the phenotype CD3–, CD16–, Leu-19+ (10). This small population (less than 5% of peripheral blood, low bouyant density lymphocytes) was isolated to greater than 95% purity using the FACS, and cultured in medium containing rIL-2 for 24 h, 1 wk, and 4 wk (Fig.7). After rIL-2 activation, CD3–, CD16–, Leu-19+ cells lysed both K562 and COLO (Fig.7). Reexamination of the antigenic phenotype indicated that greater than 95% of the cells remained CD3–, CD16–, Leu-19+. These findings show that a small subset of Leu-19+ lymphocytes lacking expression of both the CD3 and CD16 antigens can respond to rIL-2 and lyse both K562 and COLO. the relationship of these CD-3–, CD16–, Leu-19+ lymphocytes to CD3–, CD16+, Leu-19+ NK cells is unknown, although it has been suggested that this population may represent the stage of NK cell differential before acquisition of CD16 Fc receptors.

## Kinetics of the Response.

Leu 19+ T cells and NK cells were isolated from the low bouyant density lymphocyte population using the FACS, and cultured in medium containing rIL-2. Cells were harvested after 18 h and after 1 wk, then assayed for cytotoxicity against K562 and COLO. As shown in Fig. 8, killing of K562 was substantially enhanced in both the Leu-19+ T cell and NK cell populations within 18 h. Maximal lysis of COLO by NK cells was also detectable after 18 h, and did not significantly increase upon longer culture. Leu-19 T cells mediated only minimal lysis against the solid tumor cell target at all time points examined.

## Conclusions.

These studies show that the LAK phenomenon is predominately mediated by rIL-2-activated NK cells. Both the precursor and effector of this activity were typical NK cells, with the phenotype CD3–, Leu-19+. There was no evidence for a unique LAK cell. Moreover, rIL-2 apparently provided the only signal necessary for NK cells to lyse fresh solid tumor cell targets; there was no evidence that accessory cells or T lymphocytes were required to generate this response. The mechanism whereby NK cells become competent to recognize and destroy tumor cell targets after brief exposure to rIL-2 is not understood. Overall, pripheral blood T lymphocytes contributed little to the LAK phenomemon. However, these Leu-19+ T cells lysed hematopoietic targets without deliberate immunization or MHC restriction, and their cytotoxicity was usually significantly augmented by rIL-2. It has been determined that Leu-19+ T cells apparently proliferated more rapidly in response to rIL-2 than did NK cells (unpublished observation). The practical implications of this finding are that long term culture of unseparated low bouyant density peripheral blood lymphocytes in IL-2 results predominately in T cell growth, with a relative loss of NK cells. If the primary objective is to optimize generation of effectors mediating solid tumor cell cytotoxicity, this differential growth rate should be taken into account.

The studies set forth in the present invention conflict with earlier reports Grimm, E. A., A. Mazumder, H. Z. Zhang, and S. A. Rosenberg. 1982. Lymphokine-activated killer cell phenomenon. Lysis of natural killer-resistant fresh solid tumor cells by interleukin 2-activated autologous human peripheral blood lymphocytes. J. Exp. Med. 155:1823, Grimm, E. A., K. M. Ramsey, A. Mazumder, D. J. Wilson, J. Y. Djeu, and S. A. Rosenberg. 1983. Lymphokine-activated killer cell phenomenon. II. Precursor phenotype is serologically distinct from peripheral T lymphocytes, memory cytotoxic thymus-derived lymphocytes, and natural killer cells. J. Exp. Med. 157:884, Grimm, E. A., R. J. Robb, J. A. Roth, L. M. Neckers, L. B. Lachman, D. J. Wilson, and S. A. Rosenberg. 1983. Lymphokine-activated killer cell phenomenon III. Evidence that IL-2 is sufficient for direct activation of peripheral blood lymphocytes into lymphokine-activated killer cells. J. Exp. Med. 158: 884 on the LAK phenomenon. The most notable discrepancy is that earlier investigators suggested that LAK was mediated partially by a CD3+ T cell population. Recently, it has shown that a subset of low bouyant density peripheral blood T lymphocytes can lyse NK-resistant targets in the presence, but not in the absence, of mitogenic lectins Phillips, J. H. and L. L. Lanier. 1986. Lectin-dependent and anti-CD3 induced cytotoxicity are preferentially mediated by peripheral blood cytotoxic T lymphocytes expressing Leu 7 antigen. J. Immunol. 136:1579. In these early studies of the LAK effector, lectin-containing supernatant was used as the source of IL-2. Perhaps the T cell-mediated cytotoxicity in these prior studies was actually lectin-dependent cellular cytotoxicity.

In accordance with the invention, it has been shown that maximal cytotoxicity is achieved with 18 h after exposure of NK cells to rIL-2. Earlier studies suggested that this process required 3 days.

The results from this study only apply to the LAK phenomenon using lymphocytes obtained from peripheral blood. Clearly, non-MHC restricted cytotoxic lymphocytes can be generated from lymphoid tissues devoid of NK Cells, such as lymph nodes and thymus.

## SUMMARY

In vitro culture of human peripheral blood lymphocytes in IL-2 results in the generation of cytotoxic cells that can lyse fresh and cultured solid tumor cells, as well as hematopoietic tumor cell lines, without deliberate immunization or MHC restriction. This has been referred to as the lymphokine activated killer (LAK) phenomenon. In accordance with the present invention, it is shown that the majority of this activity is mediated by NK cells that express the Leu-19 (NKH-1) antigens, but do not express CD3. The precursor of this effector population also expressed the phenotype CD3−, Leu-19+. Peripheral blood CD3+ T lymphocytes contributed little to the LAK phenomenon, although low levels of non-MHC restricted cytotoxicity against hematopoietic tumor cell targets were mediated by a subset of CD3+ T lymphocytes that coexpressed the Leu-19 antigen. Further, the LAK phenomenon is not mediated by a unique LAK cell, but is mediated mainly by IL-2-activated peripheral blood NK cells.

## Claims

1. A product comprising a sterile supply of a pan-T cell monoclonal antibody and a sterile supply of a lymphokine.

2. A product according to Claim 1, wherein the lymphokine is interleukin-2.

3. A product according to Claim 1 or 2, wherein the pan-T monoclonal antibody is anti-Leu 1.

4. A product according to Claim 1, 2 or 3, which further comprises a sterile supply of a Newtonian liquid suited for bouyant density centrifugation.

5. A product according to any preceding Claim, which also includes a sealed open ended column containing sterile nylon fibers.

6. A product according to any preceding Claim, which further comprises a sterile supply of a liquid suited for density gradient centrifugation.

7. A product according to any preceding Claim, wherein the said sterile supplies are supplied in respective vials or other containers.

8. A product according to any preceding Claim, when in the form of a kit with instructions for use in treating peripheral human blood to provide an activated blood cell component for use in adoptive immunotherapy.

9. A method for treating human peripheral blood to provide an activated blood cell component for use in adoptive immunotherapy, the method comprising treating a sample of human peripheral blood to recover a viable subpopulation of white blood cells consisting substantially of natural killer cells, and incubating said subpopulation in the presence of a lymphokine to cause activation of said natural killer cells.

10. A method according to Claim 9 wherein said subpopulation of white blood cells comprises from about 60 percent to 100 percent of natural killer cells.

11. A method according to Claim 9 or 10, wherein the blood sample is treated to recover the lymphocyte cells and the lymphocyte cells are treated with a monoclonal antibody to recover said subpopulation of natural killer cells, said monoclonal antibody preferably being specific for natural killer cells or for substantially all T cells.

12. A method according to Claim 9 or 10, wherein the white blood cells are separated from the blood plasma prior to recovery of said subpopulation of natural killer cells, the granulocyte cells are separated from the white blood cells prior to recovery of said subpopulation of natural killer cells, or the granulocyte cells and the monocyte cells are separated from the white blood cells prior to recovery of said subpopulation of natural killer cells.

0257962

FIG.1

0257962

FIG.2

FIG.3

PBL - 1 wk rIL-2

FIG.4

0257962

A. Freshly Isolated

B. 1 wk rIL-2

FIG.5

0257962

A. CD5⁻, Leu-19⁺    E:T = 12:1

FIG.6

FIG.7

0257962

A. Freshly Isolated

K562 / Colo ·

○ LGL
□ CD5⁻, Leu-19+
△ CD5⁺, Leu19+

B. 18 hr rIL-2

K562 / Colo

C. 1wk rIL-2

K562 / Colo

FIG.8